# EUROPEAN PATENT SPECIFICATION

(11) **EP 1 444 890 B1**
(45) Date of publication and mention of the grant of the patent: **07.01.2009**
(21) Application number: 03002465.7
(22) Date of filing: 05.02.2003
(51) Int. Cl.: A01K 67/027, C07K 14/47, G01N 33/50, A61K 38/17, A61K 48/00, G01N 33/53, C12Q 1/68

(54) **RS1 deficient transgenic animal and uses thereof**
RS1 defizientes transgenes Tier und dessen Verwendungen
Animal transgénique présentant une déficience en RS1 et ses utilisations

(43) Date of publication of application: 11.08.2004
(73) Proprietor: Koepsell, Hermann, 97070 Würzburg (DE)
(72) Inventor: Koepsell, Hermann, 97070 Würzburg (DE); Lang, Florian, 72076 Tübingen (DE); Osswald, Christina, 97084 Würzburg (DE)
(74) Representative: Patentanwälte Ruff, Wilhelm, Beier, Dauster & Partner

(56) References cited:
- DE-A- 10 006 887
- KORN THOMAS ET AL: "The plasma membrane-associated protein RS1 decreases transcription of the transporter SGLT1 in confluent LLC-PK1 cells." JOURNAL OF BIOLOGICAL CHEMISTRY, vol. 276, no. 48, 30 November 2001 (2001-11-30), pages 45330-45340, XP002250381 November 30, 2001 ISSN: 0021-9258
- FUJITA Y ET AL: "Increased intestinal glucose absorption and postprandial hyperglycaemia at the early step of glucose intolerance in Otsuka Long-Evans Tokushima fatty rats." DIABETOLOGIA, vol. 41, no. 12, December 1998 (1998-12), pages 1459-1466, XP002250382 ISSN: 0012-186X
- THOMSON A B R ET AL: "Adaptation of intestinal nutrient transport in health and disease: Part I." DIGESTIVE DISEASES AND SCIENCES, vol. 42, no. 3, 1997, pages 453-469, XP008020466 ISSN: 0163-2116
- BOHLEN H.G.; NASE G.P.: 'Obesity lowers hyperglycemic threshold for impaired in vivo endothelial nitric oxide function' AM. J. PHYSIOL. HEART CIRC PHYSIOL. vol. 283, July 2002, pages H391 - H397
- MASON J.C. ET AL: 'Statin-induced expression of decay-accelerating factor protects vascular endothelium against complement mediated injury' CIRCULATION RESEARCH vol. 91, 18 October 2002, pages 696 - 703
- TAKADA Y. ET AL: 'Retinoschisin expression and localization in rodent and human pineal and consequences of mouse RS1 gene knockout' MOLECULAR VISION vol. 12, 28 September 2006, pages 1108 - 1116
- WIDERA A. ET AL: 'Mechanisms of TfR-mediated transcytosis and sorting in epithelial cells and applications toward drug delivery' ADVANCED DRUG DELIVERY REVIEWS vol. 55, 2003, pages 1439 - 1466
- BERNKOP-SCHNUERCH A.; PASTA M.: 'Intestinal peptide and protein delivery: novel bioadhesive drug-carrier matrix shielding from enzymatic attack' JOURNAL OF PHARMACEUTICAL SCIENCES vol. 87, no. 4, April 1998, pages 430 - 434
- RÖMPP: "Chemie Lexicon" 1989, GEORG THIEME VERLAG
- FRETZ ET AL INT. J. PHARMACEUTICS vol. 298, 2005, page 305
- SNYDER; DOWDY PHARM. RES. vol. 21, 2004, page 389

## Description

The present invention relates to a transgenic mouse homozygous for a targeted deletion of the coding region of the gene encoding the regulatory subunit (RS1) of sodium glutamate co-transporter SGLT1 chacterized in that it shows increased body weight, increased total fat and increased mean fat cell volume compared to a wild type mouse. The present invention also relates to various uses of said mouse, e.g. as an animal model for obesity or hypercholesterolaemia, for identifying substances suitable for the therapy and/or prevention of obesity or hypercholesterolaemia or for assaying the efficiency of dieting or pharmacological therapy of obesity or hypercholesterolaemia. Furthermore, the present invention relates to the use of RS1 encoding nucleic acid molecule for the treatment or prevention of diseases like obesity or hypercholesterolaemia.

In the affluent industrial nations, overnutrition (obesity) is a serious problem. The constantly rising number of persons suffering from overweight with many of them being children or adolescents is problematic due to its consequences, namely the increase in nutrition-related diseases. Overweight is a risk factor for diseases of the skeletal and musculoskeletal system, hypertension, type 2 diabetes mellitus, heart attack, breast cancer, billary stones, gout etc. Unfortunately, until now, there has not been a useful concept which could change this situation fundamentally. Too sharp a reduction of food uptake over a longer period of time is not accepted as food, which means quality of live, is always accessible and can be obtained cheaply. Until now, a change in nutrition habits by returning to a higher share of vegetable foodstuffs in one's diet is also refused. Furthermore, the known therapies are not satisfactory and have a number of side effects.

The known therapeutical forms are various special diets, partly having extreme ratios of nutrients, and drugs having side effects like steatorrhea, flatulence, diarrhoea and an increase in blood pressure.

Thus, the technical problem underlying the present invention is to provide means for the therapy and/or prevention of obesity.

The solution to said technical problem is achieved by providing the embodiments characterized in the claims. Previously, 67-68 kDa proteins from man, pig and rabbit, termed RS1 had been cloned. These proteins have about 70% amino acid identities and are involved in the regulation of SGLT1. SGLT1 is one of the two glucose transporters in the enterocytes. The Na*-D-glucose cotransporter SGLT1 is localized in the brush-border membrane and the sodium-independent glucose transporter GLUT2 in the basolateral membrance. Glucose reabsorption in small intestine is essential for energy supply by carbohydrates. The acitivities of both transporters are regulated to accommodate the glucose reabsorption to different demands. Na⁺-D-glucose cotransport activity in small intestine exhibits circadian periodicity and is regulated by carbohydrate in the diet. It is upregulated by adrenergic innervation, by insulin, glucagon-37, glucagon like peptide-2 and agiotension vasopressin, and down-regulated by cholecystokinin. The mechanisms underlying long term and short term regulations of SGLT1 in small intestine have not been resolved. Expression of SGLT1 in Yenopus laevis oocytes was used to study short term regulations and renal or small intestinal epithelial cell lines with endogenous expression of SGLT1 were employed to study long term regulations. Regulatory effects and SGLT1 have been demonstrated at the following steps: First, transcription of SGLT1 is changed by transcription factors that interact with the promoter. Second, stabiliy of SGLT1 mRNA is increased by binding of protein HuR to an uridine rich element in the 3'-untranslated region of the transporter. Third, the amount of SGLT1 in the plasma membrane may be altered by changes in exo/endocytosis. These changes are induced by protein kinases that may be activated by β-adrenergic stimulation. Fourth, the activity of SGLT1 within the membrane may be decreased by protein kinase C dependent phosphorylation.

RS1 is an intronless single copy gene that is expressed in many different tissues including small intestine. It is localized below the plasma membrane and within the nucleus and contains an ubiquitin binding associated (UBA) domain, several consensus sequences for protein kinase C and two dileucine motifs that are conserved in different species. Recently it could be demonstrated that the UBA domain of RS1 is able to bind monomeric ubiquitin.
Consistent with the dual localization of RS1, functions of SGLT1 at the plasma membrane and within the nucleus have been observed.
Coexpression experiments with RS1 and SGLT1 or other plasma membrane transporters showed that RS1 decreased the plasma membrane concentration of SGLT1 and the organic cation transporter OCT2. These effects were independent of transcription and show some not yet understood selectivity for transporters.
Regulatory effects of RS1 an SGLT1 were also demonstrated in the renal epithelial cell line LLC-PK1 that exhibits upregulation of
SGLT1 after confluence. In LLC-PK1 cells, endogeneous RS1 suppresses the transcription of SGLT1 before confluence. The data suggest that RS1 is involved in the regulation of SGLT1 and some other plasma membrane transporters and provides a link between posttranslational regulatory effects at the plasma membrane and transcriptional regulation.

In the experiments leading to the present invention RS1 knockout (RS1-/-) mice were generated that, surprisingly, showed a visceral type of obesity with 30% increased body weight, 80% increased total fat, 40% increased mean fat cell volume but unchanged food intake and motor activity. The capacity of small intestinal D-glucose uptake in RS1-/- mice was 2-fold increased compared to wild type. This was due to a 7-fold posttranscriptional upregulation of SGLT1 protein. The data suggest that an increased expression of SGLT1 in RS1-/- mice changes regulations of small intestinal functions by postprandial glucose peaks in the portal vein. These changes may result in an improved food utilization leading to obesity with increased leptin secretion by volume expanded fat cells. Thus, mice that are RS1 deficient are a valuable animal model for obesity and useful for identifying drugs for therapy of obesity.

### Brief description of the drawings

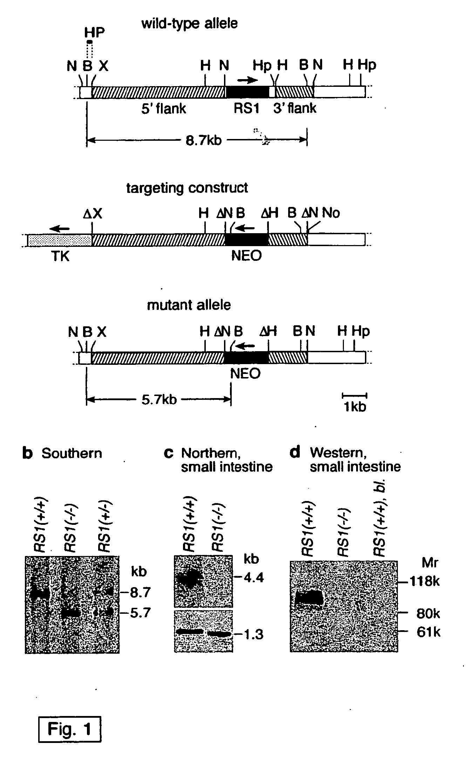

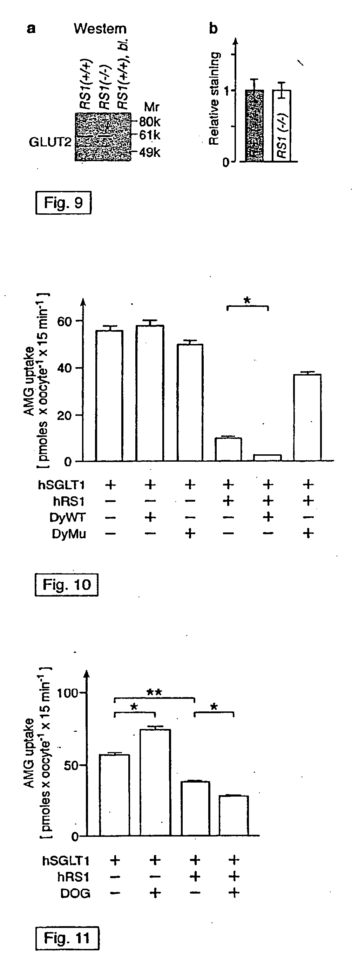

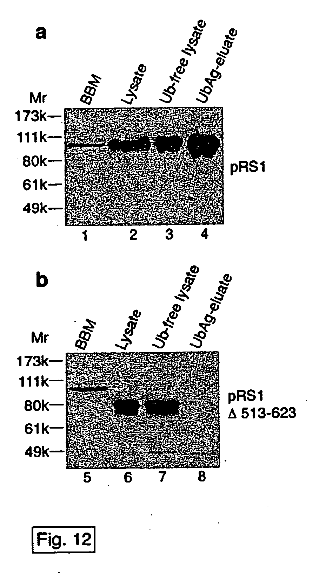

Figure 1: Targeted deletion of the coding region of the RS1 gene
   (a) the wild-type allele of the RS1 gene with the coding region (RS1), a fragment of the pPNT targeting construct with the thymidine kinase gene (TK) and the neomycin-casette (NEO), and the mutant allele are shown. Only relevant restriction sites are indicated: N, NheI; B, BamHI; X, XhoI; H, HindIII, Hp, HpI; No, NotI. The 200 bp BamHI/XhoI-fragment at the 5'-end of RS1 (HP) that was used for Southern hybridization is shown. The 8.7 kb and 5.7 kb fragments that hybridized with BamHI digested DNAs from RS1(+/+) and RS1(-/-), respectively are indicated.
   (b) Southern analysis of wild type (RS1+/+), RS1-/- and RSI-/+ mice. Genomic, BamHI-restricted DNA was hybridized with the hybridization probes indicated in Figure 1. Hybridization signals at 8.7 kb and 5.7 kb were obtained with wild type and RS1-/-, respectively.
   (c) Expression of RS1 mRNA in small intestine of wild-type and RS1-/- mice. The Northern blots were performed with 5 µg mRNA per lane and hybridized with nucleotides 934-1234 of RS1 of mouse (Genbank, Nr. Y11917). Hybridization of GAPDH was performed to control loading of the gel.
   (d) Immunodetection of RS1 in plasma membrane enriched (PME) fractions of small intestine of wild type and RS1-/- -mice using affinity-purified polyclonal antibody RS1ab against amino acids 436-454 of RS1 of mouse. Per lane 20 µg of protein of enriched plasma membrane of mouse small intestine were applied. The proteins were separated by SDS gel electrophoresis and transfered to nitrocellulose membranes. Specificity of the immune reaction with wild type PME was verified by blocking RS1ab with antigenic peptide (RS1+/+, bl.).

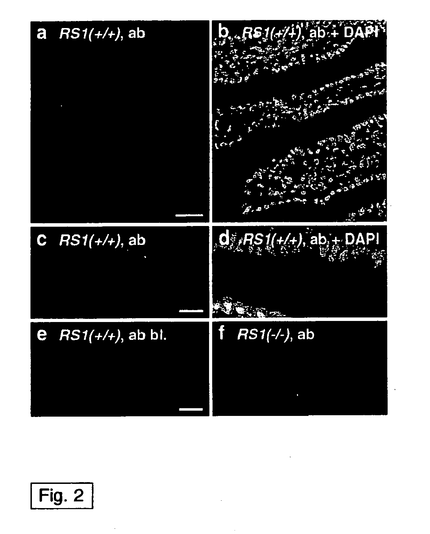

Figure 2: Immunolocalization of RS1 in small intestine of wild-type mice
   Cryosections of jejunum from RS1+/+ and RS1-/- mice were fixed with paraformaldehyde and incubated with affinity purified antibody RS1ab. The immunoreaction with affinity purified antibody against RS1 was visualized with Cy3-coupled secondary antibody against rabbit IgG-F(ab')2 (red fluorescence). In part of the immunostained sections the nuclei were stained with DAPI. Immunoreactions with wild type are shown in a,b,c,d,e, and with RS1-/- in f. In e, the immunoreaction was blocked with antigenic peptide. In f, the non-blocked immunoincubation of a section of small intestine of a RS1-/- mouse is shown. The left panel shows the localization of RS1 by red fluoresence of Cy3-coupled secondary antibody. On the right panel the nuclear localization of RS1 is demonstrated by colocalization of red Cy3-fluorescence of RS1ab and green DAPI-staining of the nuclei. The blue fluorescence of DAPI was converted to green to demonstrate the colocalizations in yellow. Bars: (a) 20 µm; (c,e) 5 µm.

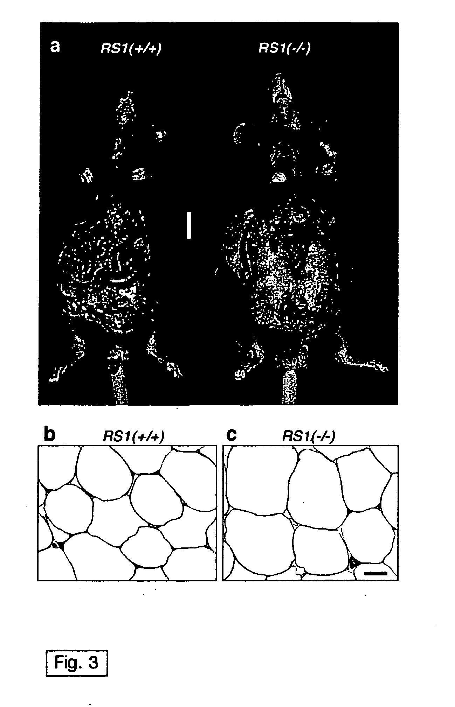

Figure 3: Phenotype of RS+/+ and RS1-/- mice
   (a) Photos of 5 months-old male mice originating from identical litters. Scale 1 cm. The RS1-knockout mouse has much more fatty tissue in the abdomen and the fat cells have an enlarged size.
   (b),(c) Light-micrograph of fatty tissue from the abdomen of RS1+/+ and RS1-/- mice. Scale 30 µm. Based on the area of 500 fat cells it has been calculated that the volume of the fat cells of the RS1-/- mice is 40 +/- 6% higher than of wildtype mice.

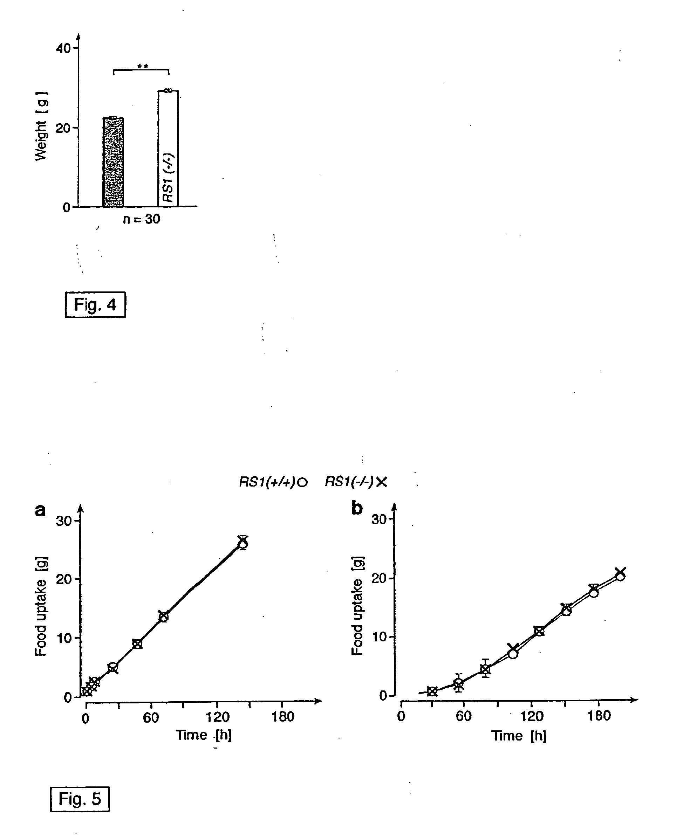

Figure 4: Weight Comparison of RS1+/+ and RS-/- mice
   The weights of 5 months-old female RS1+/+ and RS1-/- mice from identical cross-breedings of homogygotic RS1-/- mice with BalbC57BL/6J mice have been determined. The animals have been grown under standard conditions. Mean values (+/- standard deviation from the mean value) of 30 animals have been shown. The student t-test showed that the body weight of RS1-/- mice had been significantly higher than that of the RS+/+ mice (**P>0.001).
Figure 5: Food uptake and body weight increase
   Groups of 5 male and 5 female RS1+/+ and RS1-/- mice from identical cross-breedings of RS1-/- mice with RS1+/+ BalbC57BL/6J mice were compared. All mice were 5 months old. In both feeding experiments water was supplied ad libitum. In (a) standard dry food was also supplied ad libitum whereas in (b) standard dry food ad libitum was only supplied for 8 h during the day. Linear regression analysis was performed an the curves in (a) and an the straight portions of the curves in (b) obtained after adaption (days 4 to 9). The cumulative food uptake of the RS1+/+ mice (°) and the RS1-/- mice (x) has been shown. For RS1+/+ and RS1-/- nearly identical slopes were obtained. The slopes in (b) were significantly smaller that those in (a) (P< 0.01 ). However, both feeding conditions did not show any difference with regard to food uptake by RS1-/- mice compared to RS1+/+ mice.

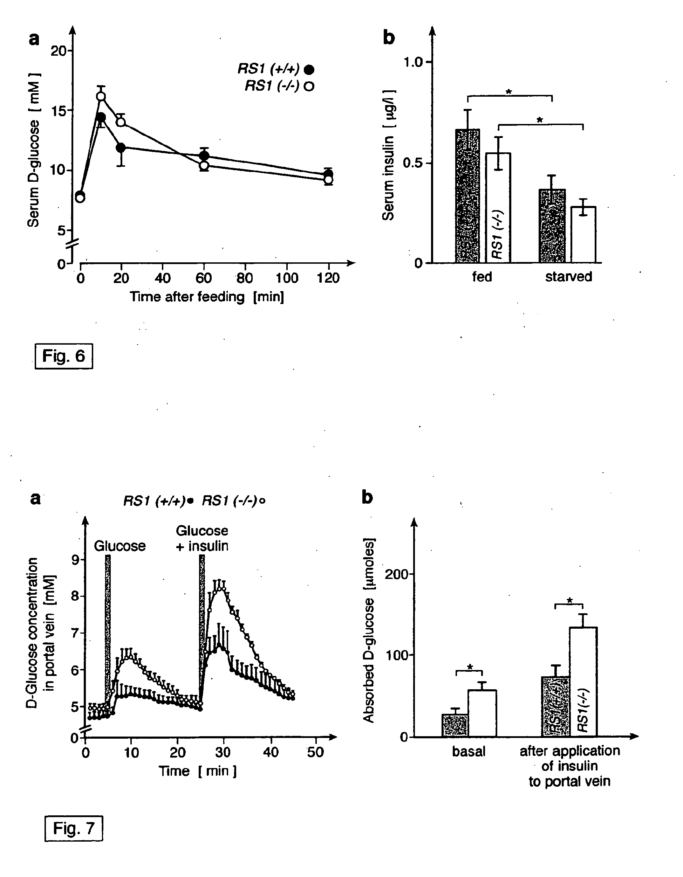

Figure 6: Glucose tolerance and absorption of D-glucose in small intestine
   (a)Glucose tolerance test was performed in 4-6 five months old RS+/+ and RS-/- mice. Each group contained 10 animals from identical litters. A 1.1 molar solution containing 2.5 mg D-glucose per g body weight was applied into the esophagus. Serum concentrations of D-glucose were measured before and after D-glucose application and are presented (n=10, equally mixed gender). The results are mean values +/- standard deviations. It has been shown that the differences in the serum glucose concentrations have not be statistically significant.
   (b)The serum insulin concentration was measured in the morning in RS+/+ mice (grey columns) and RS1-/- mice (open columns). A part of the animals had free access to the dry food (fed group) whereas the other group received 24 hours no food (starved group). Per group 20 RS1+/+ mice and 20 RS-/- mice from identical litters were analysed. Mean values +/- standard deviations are given. The insulin concentrations of RS1+/+ and RS1-/- mice in the starving groups were significantly lower than the insulin concentrations in the fed groups (*P<0.05). The data show that the insulin dependent regulation of the serum glucose concentration in the RS1-/- mice compared to the RS1+/+ mice has not been significantly changed.
Figure 7: Resorption of D-glucose from the small intestine and liver of RS1+/+ and RS1-/- mice
   Measurements of D-glucose absorption in perfused total small intestine are shown in (a) and (b). They were performed with an ex vivo preparation of small intestine and liver that were perfused through the respective arteries. At the indicated times 100 mg D-glucose (glucose) dissolved in 0.25 ml saline were infused into the duodenum. The D-glucose concentrations in the portal outflow before and after duodenal D-glucose application are shown in (a). During the second D-glucose application insulin was applied to the liver via the portal vein. (b) shows the total amounts of absorbed D-glucose that were calculated from the increase of D-glucose concentrations in the portal outflow and the volume of the outflow. (n=5, male). It has been shown that the amount of the resorbed glucose has been significantly higher in the RS1-/- mice than in the RS1+/+ mice (P*<0.05). It appears that the observed obesity in RS1-/- mice is caused by the increased glucose resorption in small intestine.

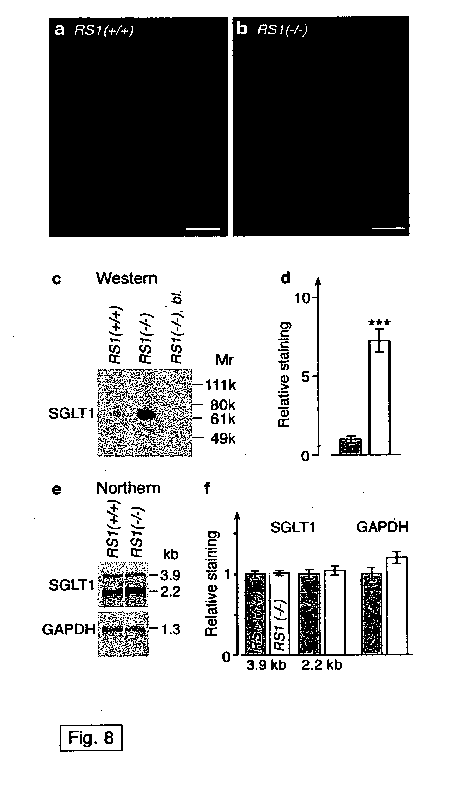

Figure 8: Expression of the Na⁺-D-glucosecotransporter SGLT1 in small intestine of RS1-/- mice compared with RS1+/+ mice
   Cryosections of jejunum from RS1+/+ (a) and RS1-/- mice (b) were fixed with paraformaldehyde, reacted with the affinity-purified antibody SGLT1 ab (1:200) and developed with a Cy3-coupled secondary antibody. The sections were also stained with DAPI. The fluorescence was visualized by light microscopy. Bars: 10 µm. For (c) - (f) from small intestines of 10 RS1+/+ or 10 RS1-/- 5 months old female mice plasma membrane enrichted (PME) fractions containing luminal and basolateral plasma membranes of the enterocytes and mRNAs were prepared. © Western blot with PME fractions from RS1+/+ and RS1-/- that were developed with SGLT1 ab. Per lane 2.5 µg of protein were applied. The reaction with SGLT1 ab could be blocked with antigenic peptide (RS1-/-, bl). A densitometric quantification of 6 reactions from two independent experiments is shown in (d).
   (e) Northern blots that were developed with specific cDNA probes for SGLT1 or GAPDH. Per lane 5 µg of mRNA were applied. In (f) 6 reactions from 3 independent experiments were quantified.
Figure 9: Comparison of the protein amount of the natrium-independent glucosetransporter GLUT2 in plasma membranes of small intestine epithelial cells of RS1-/- and RS1+/+ mice
   (a) Western blots that were developed with an antibody against GLUT2. 10 µg protein of the PME fractions shown in Fig. 8 were applied per lane. In the right lane, the antibody was blocked with the antigenic peptide. (b) 4 reactions from 2 independent experiments were quantified.
Figure 10: Coexpression of SGLT1 and RS1 in oocytes of *Xenopus laevis* with and without simultaneous expression of intact dynamin or a dominant negative dynamin mutant
   The indicated combinations of hSGLT1-cRNA (2.5 ng), hRS1-cRNA (10 ng), wild-type dynamin (DyWT, 10 ng) and dominant negative dynamin mutant (DyMu, 10 ng) were injected into oocytes of Xenopus laevis and the expressed uptake of 50 µM [¹⁴C]AMG measured after three days. Mean values +/- SEM of 6-10 oocytes are indicated. *P < 0.05
Figure 11: Coexpression of SGLT1 and RS1 in oocytes of *Xenopus* laevis without and with activation of protein kinase C
   2.5 ng hSGLT1-cRNA without and with 10 ng hRS1-cRNA were injected into oocytes of Xenopus laevis. After three days incubation for expression either 50 nl Ori-buffer or 50 nl Ori-buffer containing 20 µM sn-1,2-dioctanoyl glycerol (DOG) were injected. After 30 min. incubation the expressed uptake of 50 µM [¹⁴C] AMG was measured. Mean values +/- SEM of 6-10 oocytes are indicated. *P < 0.05, **P < 0.01
Figure 12: Non-covalent binding of ubiquitin monomer to pRS1
   PRS1 or pRS1Δ513-623 lacking the UBA domain were expressed in HEK293 cells. The cells were lysed (lysate), free ubiquitin was removed (UB-free lysate) and the ubiquitin free lysate was incubated with agarose beads with covalently linked monoubiquitin. After washing, the proteins bound to the beads were eluted with SDS and separated by polyacrylamide gel electrophoresis. The proteins were blotted to nitrocellulose and developed with affinity-purified antibody against pRS1. Brush border membranes (BBM) isolated from proximal tubules of pig kidney were used as control.

Thus, in a first embodiment, the present invention relates to a transgenic mouse homozygous for a targeted deletion of the coding region of the gene encoding the regulatory subunit (RS1) of sodium glutamate co-transporter SGLT1 characterized in that it shows increased body weight, increased total fat and increased mean fat cell volume compared to a wild type mouse. As used herein, the term "RS1-/-" refers to any mice wherein (a) both alleles encode an inactive RS1 version which has, e.g., completely or partially lost its capability to bind monomeric ubiquitin and/or to regulate transcription of SGLT1, These RS1 versions comprise RS1 molecules containing substitution(s), deletion(s) and/or insertions of one or more amino acids rendering the molecule The person skilled in the art can generate such versions of RS1 using the wild type protein or nucleic acid sequence (disclosed in Lambotte et al., DNA and Cell Biology 15 (1996), 769-777; Reinhardt el., Biochem.Biophys.Acta 1417 (1999), 131-143; Veyhl et al., J.Biol.Chem. 268 (1993), 25041-15053) as starting material. By means of conventional molecular biological processes (see, e.g., Sambrook et al., Molecular Cloning, A Laboratory Manual 2nd edition (1989) Cold Spring Harbor Laboratory Press, Cold Spring Harbor, NY.) different mutations can be introduced into the wild type nucleic acid molecules. As a result, RS1 molecules with the desired modified biological properties are synthesized. One possibility is the production of deletion mutants in which nucleic acid molecules are produced by continuous deletions from the 5'- or 3'-terminal of the coding DNA sequence and that lead to the synthesis of polypeptids that are shortened accordingly. Another possibility is the introduction of single-point mutation(s) at positions where a modification of the amino acid or deletion of amino acid sequences influences particular properties sequence (e.g., phosphorylation, ubiquiting binding (with the UBS being located from aa 550 to aa 576; Genbank accession No. Y11917 etc.). By these methods versions of RS1 can also be produced, for example, that are no longer subject to the regulation mechanisms that normally exist in the cell, e.g. with regard to allosteric regulation or covalent modification. Such forms of RS1 might also be valuable as therapeutically useful antagonists of biologically active RS1. The person skilled in the art can easily determine an altered expression of the RS1 encoding gene, e.g., by Northern blotting as described in Example 1(F), RT-PCR etc., as well a reduced or inhibited biological activity of the RS1 protein, e.g., by phenotype analysis of RS1-/- mice (see Examples 1 and 5), determinations of D-glucose and lipids in serum of RS1-/- mice (see Examples 1 and 7), determination of serum glucose and glucose reabsorption in small intestine (see Examples 1 and 8), determination of expression of SGLT1 (and GLUT2) in small intestine (see Examples 1 and 9), determination of binding of monomeric ubiquitin to RS1 (c.f. Example 13).

For the manipulation in prokaryotic cells by means of genetic engineering the RS1 encoding nucleic acd molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis methods usually sequence analysis, restriction analysis and other biochemical or molecular biological methods are used.

The provision of the altered nucleic acid molecules described above opens up the possibility to produce transgenic RS1-/-mice. Techniques how to achieve this are well known to the person skilled in the art. Such methods, e.g., comprise the introduction of a nucleic acid molecule or recombinant vector, preferably pPNT and variants thereof. (Tybulewicz et al., Cell 65, 1151-1163 (1991)) containing a modified RS1 gene (coding and/or regulatory region), antisense or ribozyme encloding DNA etc., into a germ cell, an embryonic cell, stem cell or an egg or a cell derived therefrom. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe; see also Example 1C-F, below.

In a preferred embodiment the transgenic mouse comprises a nucleic acid molecule or corresponding vector as described above, preferably wherein said nucleic acid molecule or vector is stably integrated into the genome of said non-human animal, preferably such that the presence of said nucleic acid molecule or vector leads either to partially or completely blocked gene expression or to expression of an (completely or partially) inactive RS1. Said mouse may have one or several copies of the same or different nucleic acid molecules encoding one or several mutant forms of RS1 polypeptide. This mouse has numerous utilities and therefore, presents a novel and valuable animal model in the development of therapies, treatment, etc. for diseases caused by aberrant expression/activity of a RS1, e.g., obesity, hypercholesterolaemia, type II diabetes etc.

Preferably, the transgenic mouse of the invention is a mouse, wherein the gene encoding RS1 contains a deletion within its coding region resulting in the production of an inactive truncated protein or in a frame shift. Particularly preferred is a transgenic mouse, wherein substantially the entire coding region of the gene encoding RS1 has been deleted.

Alternatively, the expression of RS1 can be modified, i.e. reduced or eliminated. This can be achieved by various approaches, e.g. by introducing mutations into regulatory sequences, e.g., promoter sequences. Alternatively, the reduction of expression can be achieved by an anti-sense-, sense-polynucleotide, ribozyme, co-suppression and/or dominant mutant effect. "Antisense-polynucleotides" means DNA or RNA constructs which block the expression of the naturally occurring gene product.

In summary, the transgenic mice of the present invention may be characterized by the following feature:
(a) disruption or (partial) elimination of the endogenous alleles encoding RS1 (coding and/or regulatory regions).

All the applications that have been herein before discussed with regard to a transgenic mouse also apply to mice carrying two, three or more transgenes. It might be also desirable to induce inactive RS1 (and/or wild type RS1) expression or function at a certain stage of development and/or life of the transgenic mouse. This can be achieved by using, for example, tissue specific, developmental and/or cell regulated and/or inducible promoters for controlling RS1 expression. A suitable inducible system is for example tetracycline-regulated gene expression as described, e.g., by Gossen and Bujard (Proc. Natl. Acad. Sci. 89 US (1992), 5547-5551) and Gossen et al. (Trends Biotech. 12 (1994), 58-62). In order to achieve expression only in a desired target organ, the RS1-/- encoding nucleic acid molecules can be linked to a tissue specific promoter. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

The transgenic mice of the invention are well suited for, e.g., pharmacological studies of drugs.

Thus, in a further embodiment, the present invention relates to the use of a transgenic mouse of the invention for identifying substances suitable for the therapy and/or prevention of obesity or hypercholesterolaemia. Such substances can be screened by a method comprising the steps of:
(a) administering a candidate compound to the transgenic mouse of the invention; and
(b) determining whether said compound results in (i) decreased body weight, decreased total fat and decreased mean fat volume.

Assays for determining one or more of the parameters of (b) are well known to the person skilled in the art and also described in the Examples. Alernatively (or additionally) the change of serum cholesterol concentration (see Example 1(N)), or SGLT1 activity (see Examples 8 and 9) can be measured.

Administration of the candidate compound may be effected by different ways, e.g. by intravenous, intraperetoneal, subcutaneous, intramuscular, topical or intradermal administration. The route of administration, of course, depends on various factors, e.g. the kind of compound. The candidate molecule can be rationally designed using known techniques. Alternatively, the candidate compounds may be obtained from expression libraries, e.g., cDNA expression libraries, and may be peptides, proteins, nucleic acids, antibodies, small organic compounds, ligands, hormones, peptidomimetics, PNAs or the like. The therapeutically useful compounds isolated by the above methods/assays also serve as lead compounds for the development of analog compounds.

Any of the above assays is also useful for determining the efficiency of therapeutic measures. Thus, in a further embodiment, the present invention also relates to the use of the transgenic mouse of the invention for assaying the efficiency of dieting or pharmacological therapy of obesity or hypercholesterolaemia.

The present invention also relates to the use of a RS1 encoding nucleic acid molecule for the preparation of a pharmaceutical composition for treatment of obesity or hypercholesterolaemia.

The delivery of nucleic acid molecules encoding RS1 (or the antisense RNAs or ribozymes) can be achieved by direct application or, preferably, by using a recombinant expression vector such as a chimeric virus containing these compounds or a colloidal dispersion system. Direct application to the target site can be performed, e.g., by ballistic delivery, as a colloidal dispersion system or by catheter to a site in artery. The colloidal dispersion systems which can be used for delivery of the above nucleic acid molecules include macromolecule complexes, nanocapsules, microspheres, beads and lipid-based systems including oil-in-water emulsions (mixed), micelles, liposomes and lipoplexes, The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods).

Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957. In order to achieve expression only in the desired target organ, the nucleic acids encoding, e.g. RS1 or an antisense RNA or ribozyme can also be operably linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

The present invention also relates to methods for the diagnosis of obesity or hypercholesterolaemia or of a risk for developing said diseases.

In a first embodiment, the present invention provides a method for the diagnosis of obesity or hypercholesterolaemia or of a risk for developing said diseases, comprising the following steps:
(a) contacting a sample with an anti-RS1-antibody capable of specifically binding to RS1 or a probe comprising at least one oligonucleotide capable of specifically hybridizing to RS1 DNA or mRNA; and
(b) determining the concentration and/or sequence of RS1 or RS1 DNA or mRNA;
wherein a decreased concentration of RS1 or RS1 mRNA or the presence of a mutation within the RS1 DNA or mRNA sequence resulting in an RS1 protein with complete or partial loss of activity is indicative for obesity or hypercholesterolaemia or for a risk for developing said diseases, compared to healthy individuals.

The decrease of the concentration of RS1 or RS1 mRNA can be oberserved if the gene encoding RS1 contains a deletion within its coding region resulting in the production of an inactive truncated protein or in a frame shift. In a particularly preferred case substantially the entire coding region of the gene encoding RS1 has been deleted.

The expression of RS1 can be also modified, i.e. reduced or eliminated. This can be achieved by various approaches, e.g. by introducing mutations into regulatory sequences, e.g., promoter sequences. Alternatively, the reduction of expression can be achieved by an anti-sense, sense-polynucleotide, ribozyme, co-suppression and/or dominant mutant effect. "Antisensepolynucleotides" means DNA or RNA constructs which block the expression of the naturally occurring gene product.

Techniques for carrying out the diagnostic method of the invention are well known to the person skilled in the art. When the target is the RS1 protein, the probe is an anti-RS1-antibody probe. The term "antibody", preferably, relates to antibodies which consist essentially of pooled monoclonal antibodies with different epitopic specificities, as well as distinct monoclonal antibody preparations. Monoclonal antibodies are made from an antigen containing a fragment of the proteins of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of speicifically binding to the protein. Fab and F(ab')2 fragments lack the Fc fragment of intact antibody, clear more rapidly from the circulation, and may have less non-specific tissue binding than an intact antibody. "Wahl et al., J. Nucl. Med. 24:316-325 (1983).) Thus, these fragments are preferred, as well as the products of a FAB or other immunoglobulin expression library.

According to the invention the probe (a) is an anti-RS1-antibody or (b) comprises at least one oligonucleotide capable of specifically hybridizing to RS1 DNA or mRNA. Ther term "probe" of (b) also includes primers which can be used in a polymerase chain reaction, ligase chain reaction etc.. Preferably, said oligonucleotides have a length of at least 10, in particular of at least 15 and, particularly preferred, of at least 50 nucleotides.

In a particularly preferred embodiment of the diagnostic method of the invention, the probe (e.g. antibody or oligonucleotide used for determining the concentration of RS1 mRNA by hybridization assays) is detectably labelled, for example, with a radioisotope, a bioluminescent compound, a chemiluminescent compound, a fluorescent compound, a metal chelate, or an enzyme. A variety of techniques are available for labelling biomolecules, are well known to the person skilled in the art and are considered to be within the scope of the present invention. Such techniques are, e.g., described in Tijssen, "Practice and theory of enzyme immuno assays", Burden, RH and von Knippenburg (Eds), Volume 15 (1985), "Basic methods in molecular biology"; Davis LG, Dibmer MD; Battey Elsevier (1990), Mayer et al., (Eds) "Immunochemical methods in cell and molecular biology" Academic Press, London (1987), or in the series "Methods in Enzymology" Academic Press, Incl. There are many different labels and methods of labelling known to those of ordinary skill in the art. Commonly used labels comprise, inter alia, fluorochromes (like fluorescein, rhodamine, Texas Red, etc.), enzymes (like horse radish peroxidise-galactosidase, alkaline phosphatase), radioactive isotopes (like 32P or 125I), biotin, digoxygenin, colloidal metals, chemi- or bioluminescent compounds (like dioxetanes, luminol or acridiniums). Labeling procedures, like covalent coupling of enzymes or biotinyl groups, iodinations, phosphorylations, biotinylations, random priming, nicktranslations, tailing (using terminal transferases) are well known in the art. Detection methods comprise, but are not limited to, autoradiography, fluorescence microscopy, direct and indirect enzymatic reactions, etc.

For determining the sequence of the RS1 encoding gene well known methods, e.g. Sanger sequencing, may be used.

In a further embodiment, the present invention provides a method for the diagnosis of obesity or hypercholesterolaemia or of a risk for developing said diseases, comprising the following steps:
(a) determining the nucleic acid sequence of the RS1 gene from a sample obtained from an individual; and
(b) comparing the sequence obtained with the nucleic acid sequences of the RS1 gene obtained from healthy individuals and individuals having obesity or hypercholesterolaemia;
wherein a nucleic acid sequence corresponding to the nucleic acid sequence of individuals with obesity or hypercholesterolaemia is indicative for obesity or hypercholesterolaemia or for a risk for developing said diseases. Preferably, said method is based on a collection of statistically analysed RS1 mutations/polymorphisms found in patients afflicated with obesity or hypercholesterolaemia wherein the RS1 gene sequence of a sample is compared with the collection of RS1 mutations/polymorphisms observed in patients having obesity or hypercholesterolaemia.

For use in the diagnostic research discussed above, kits are described. Such kits contain (a) probe(s) as described above and are useful for carrying out the diagnostic methods of the invention. The probe can be detectably labeled. Such probe may be a specific antibody or specific oligonucleotide. Said kit may contain an anti-RS1-antibody and allows said diagnosis, e.g., by ELISA and may contain the antibody bound to a solid support, for example, a polystyrene microtiter dish or nitrocellulose paper, using techniques known in the art. Alternatively, said kits are based on a RIA and contain said antibody marked with a radioactive isotope. In addition the antibody in the kit may be labeled with enzymes, fluorescent compounds, luminescent compounds, ferromagnetic probes or radioactive compounds. The kit may comprise one or more containers filled with, for example, one or more probes.

The following examples illustrate the invention.

### Example 1

### General methods

### (A) Mice

Mice were housed and handled according to institutional guidelines complying with German legislation. In all experiments the compared animals, RS1-/- and wild-type mice, were of the same mixed genetic background (H129 and C57BL/6J) and between 4 and 6 months of age. Comparison of RS1-/- and wild-type mice was always performed with animals from the same litters that were obtained after crossing back with C57BL/6J between the 4.-10. generation as indicated. Animals were kept in a temperature-controlled environment with a 12-h light/12-h dark cycle. The received the altromin standard diet (Altromin GmbH, Large-Lippe, Germany) and water ad libitum. Blood and tissue samples were taken and weight determination were performed between 10.00 and 11.00 a.m.

### (B) Materials

Peroxidase-conjugated goat anti-rabbit IgG antiserum, alkaline phosphatase-coupled goat anti-rabbit Ig F(ab')2 antiserum, and m-maleimidobenzoyl-N-hydroxy-succimide were obtained from Sigma (München, Germany). Indocarbocyanin (Cy3)-conjugated goat anti-rabbit IgG-F(ab')2 antiserum was delivered by Dianova GmbH (Hamburg, Germany), phloretin by Fluka (Neu-Ulm, Germany), and human insulin (>27.5 USP units per mg) by ICN Biochemicals (Meckenheim, Germany). Affinity purified antibody against the sodium-independent glucose transporters GLUT2 (from rat that cross-reacts with GLUT2 from mouse) including the antigenic peptides was obtained from Alpha Diagnostic International (San Antonio, USA). ELISA for quantification of insulin of rat or mouse was supplied by CrystalChem (Chicago, USA). Prestained molecular weight markers for SDS polyacrylamide gel electrophoresis were obtained from Invitrogen (Groningen, Netherlands).

[¹⁴C] Labeled methyl-α-D-glucopyranoside (AMG) containing 5.7 GBq/mmole), the restriction enzymes, sn-1,2-dioctanoyl glycerol (DOG), and all other materals were obtained as described earlier (Lambotte et al., (1996) DNA Cell Biol. 15, 769-777; Mehrens et al. J. Am. Soc. Nephrol. 11, 1216-1224).

### (C) Cloning of mouse RS1 genomic DNA and construction of the replacement targeting vector

Two degenerate primers were designed based an the nucleotide sequence of pig, rabbit and human RS1 cDNA: 5'-AATCC(GC)(CT)T(AG)ATGGAAGT(AG)GA-3' (forward, position 1099-1118 on pig RS1, accession number X64315) and 5'-GCTTCCTGCAA(AG)GT(AG)AAGCC-3' (reverse, position 2027-2008 on pig RS1). The PCR was done with these primers using mouse kidney cDNA as a template (33 cycles 94° 30sec, 50° 1 min, 72° 1 min with 2 sec extension every cycle). The amplificate was cloned and the sequencing revealed the high similarity to the known RS1 genes. The cloned RS1 fragment was used to screen the BAC mouse genomic library (Genome Systems, St.Louis, USA). The positive clone was analysed using restriction analysis and Southern blotting, the RS1 gene with 5'- and 3'-flanking regions was localized an the 120 kb insert and completely sequenced an both strands (the sequence was submitted to Genebank, accession number Y11917). To create the replacement target vector at first the 3'-flanking region of RS1 (1,5 kb HindIII/NheI fragment) was cloned into the filled NotI/XhoI sites of the vector pPNT (Tybulewicz et al., Cell 65 (1991), 1153-1165) and then the 5'-flanking region of RS1 (5,4 kb XhoI/NheI fragment) was inserted into the KpnI site of this vector treated with mung bean nuclease. The replacement vector was verified by restriction analysis and sequencing of the vector-insert boundaries. The target vector shown in Fig. 1 lacks the complete RS1 coding region and contains 5'- and 3'- flanking regions of mouse RS1 in the wild-type orientation separated by a neomycin resistance gene that was introduced in opposite direction.

### (D) Southern analysis and genomic PCR

Genomic DNA was digested with BamHI and hybidized with a 200 bp BamHI/XhoI-fragment from the 5'-end of RS1. The hybridization probe and the fragments hybridizing with RS1+/+ and RS1-/- are shown in Figure 1. For genomic typing by PCR, primers were derived from the noncoding 3'-end of RS1 (P1, 5'-CCCCACACCCTTCCCATGGTCATGA-3', reverse, position 2367-2391), the open reading frame of RS1 (P3, 5'-GGGAATGCAGACCTTGCCCTTCTTG-3', forward, position 1689-1713) and the neomycin casette of the pPNT vector (P2, 5'-CCACTTGTGTAGCGCCAAGTGCCAG-3', reverse, position 93-117).

### (E) Transfection and selection of recombinant ES cells

The pPNT based targeting vector depicted in Figure 1 was linearized by NotI and embryonic stem cells (strain H 129) were transfected by electroporation. Recombinant cells were selected by geniticin G-418 and analyzed by Southern hybridization and genomic PCR. Cells with recombinant alleles were introduced in blastocytes of C57BL/6J cells.

### (F) Northern analysis

Northern blotting was performed according to standard procedures with 5 µg mRNA per sample using radioactively labeled polynucleotide probes (Korn et al., J.Biol.Chem. 276 (2001), 45330-45340). Poly (A+)mRNA was isolated from total RNA by oligo-dT affinity purification using the "Oligote mRNA spin column"-kit from QIAGEN GmbH (Hilden, Germany). The following polynucleotide probes were used for hybridization: mouse RS1 (nt 934-1234, GenBank accession no. Y11917), mouse SGLT1 (nt 1-315, GenBank accession no. AF163846), mouse GLUT2 (nt 1580-1863, GenBank accession no. X15684, and human glyceralhehydephosphate dehydrogenase GAPDH (1.1 kb fragment from CLONTECH, Heidelberg, Germany).

### (G) Generation and purification of antibodies

Antibodies against peptides of mouse RS1 (RS1 ab, amino acids 436-454, GLSPDREDVRRSTESARKS) and mouse SGLT1 (SGLT1 ab, amino acids 586-601, KDTIEIDTEAPQKKKG) were prepared in rabbits as described (Meyer-Wentrup et al., Biochem.Biophys.Res.Commun. 248 (1998), 673-678). The peptides were synthesised with a cystein residue at the C-terminus. For immunization they were coupled to ovalbumin using m-maleimidobenzoyl-N-hydroxysuccimide ester. The titers of the antisera were determined by ELISA using the antigenic peptides as antigens and alkaline phosphatase-coupled goat anti-rabbit Ig F(ab')2 as secondary antibody. Affinity purification of the antisera was performed an the antigenic peptides coupled polyacrylamide beads using the Sulfolink Kit from Pierce (Bonn, Germany).

### (H) SDS-polyacrylamide gel electrophoresis and Western blot analysis

Generally: For SDS polyacrylamide gel electrophoresis, protein samples were incubated for 1 h at 37°C in 50 mM Na₂HPO₄ pH 6.8, 4 M urea, 0.25 M β-mercaptoethanol, 1% (w/v) SDS and 0.0005 % (w/v) Bromphenol blue. SDS polyacrylamide gel electrophoresis and Western blotting was performed as described earlier (Korn et al. (2001) J. Biol. Chem. 276, 45330-45340). As primary antibodies against pRS1 we used the previously characterized antibody pRS1-ab (Korn et al. (2001) J. Biol. Chem. 276, 45330-45340) that was raised in rabbits against expressed pRS1 and was affinity-purified. As secondary antibody, peroxidase-conjugated goat anti-rabbit IgG antiserum from Sigma (1: 5000 dilution) was used.
Specific proteins: A cell homogenate was prepared from fat tissue and a plasma membrane-enriched fraction (PME) from small intestine. Both tissues were frozen in liquid nitrogen, pulverized in the frozen state, suspended in 20 mM Tris-HCl, pH 7.5, 250 mM saccharose, 5 mM EGTA, 5 mM MgS04 plus protease inhibitors (Korn et al., 2001, J. Bio. Chem. 276, 45330-45340), and homogenzied at 4°C with a glass teflon homogenizer. After cell debris was removed by a 10-min centrifugation at 150 x g the cell homogenate was obtained. To prepare the PME fraction from small intestine the homogenate was centrifuged 10 min at 2000 x g, the obtained supernatant was centrifuged 60 min at 40,000 x g, and the PME fraction collected as pellet. Brush-border membranes from renal proximal tubules were isolated by a Ca2+ precipitation method (Koepsell and Seibicke, Methods in Enzymology 191 (1990), 583-605). Protein concentrations were determined using the Bradford protein assay (Bio-Rad Laboratories, München, Germany). SDS polyacrylamide gel electrophoresis, Western blotting and immunodetection were performed as described earlier (Valentin et al., Biochem. Biophys. Acta 1468 (2000), 367-380) with the exceptions that urea was omitted from the sample buffer and that incubation with primary antibodies was performed 16 h at 4°C. To test the specificity of the immunoreactions the primary antibodies were incubated for 1 h at 37° C with 100 µg/ml of antigenic polypeptide before they were added to the blot

### (I) Immunohistochemistry

Small intestine, fat tissue or kidney from mice were rapidly frozen in liquid isopentane that was cooled in liquid nitrogen and sectioned in a cryostat. 5 µm-thick cryosections were thawed an silanized glass slides and fixed by 5-min incubation at room temperature with PBS containing 4% (w/v) paraformaldehyde. The sections were washed for 15 min with PBS containing 0.05% (w/v) Tween 20 (PBS-T) and nonspecific binding sites were blocked by 30 min-incubation with PBS containing 0.1% (w/v) Triton X-100 plus 2% (w/v) skim-milk powder (blocking buffer). Incubation with affinity-purified primary antibodies dissolved in blocking buffer was performed for 16 h at 4°C. The sections were washed three times with PBS-T and bound antibody was detected by incubation for 1 h at room temperature in the same buffer containing 1: 2000 diluted goat anti-rabbit IgG antibody labeled with indocarbocyanin (Cy3) from Dianova (Hamburg, Germany). After washing with PBS-T the sections were embedded in fluorescent mounting medium supplied by DAKO Diagnostik GmbH (Hamburg, Germany) that contained 4',6'-diamidino-2-phenylindole (DAPI) for staining of nuclei. To test the immunohistochemical reactions for specificity 100 µg/ml of the respective antigenic peptides were added to the primary antibody solution and incubated for 60 min at 37°, before they were applied to the sections. Light microscopic and Laser scanning fluorescence microscopy were performed with the Axiphot 2 microscope and the confocal microscope LSM 510 from Zeiss using the recommended filter combinations and wave lengths.

### (J) Determinations of weight and body composition

To determine total fat mass, water content and dry mass, killed mice were weighted, dried individually for five days at -90°C and weighted again. For lipid extraction (Halaas et al., Science 269 (1995), 543-546) each dried mouse was homogenized with 300 ml of chloroform/methanol (3:1). The lipid-free dry tissue was removed by filtration through filter paper (Schleicher & Schuell, Dassel, Germany), and the filters were dried and weighted.

### (K) Electronmicroscopy of fat cell and quantification of cell size

Fat tissue was fixed with glutaraldehyde (2.5%(v/v)), formaldehyde (2%(v/v)) and OsO4 (2%(w/v)), embedded in Epon 812 (Serva, Heidelberg, Germany), semithin (0.5 µm sectioned), examined by electron microscopy and photographed. The relative section areas of fat cells in RS1+/+ and RS1-/- mice were determined by counting section points of fat cell borders with an overlaid grid. For a comparison of fat cell volumes the relative section areas in RS1-/- compared to wild-type cells were transformed to relative volumes by assuming an idealized spherical shape of the fat cells.

### (L) Analysis of feeding behaviour

These experiments were performed with five months old, female or male mice that obtained water ad libitum. Ingested food and body weight were determined daily. In one protocol the animals were continuously supplied with standard diet ad libitum. In another protocol the animals were starved over night and food ad libitum was supplied from 9 a.m to 5 p.m.

### (M) Analysis of motoric activity

Motoric activity was determined in cages with running wheels in which the revolutions were counted independent of direction.

### (N) Analysis of D-glucose and compounds of lipid metabolism in serum

D-glucose was determined with glucose dehydrogenase (Banauch et al., Zeitschrift für klinische Chemie und klinische Biochemie 13 (1975), 101-107). Concentrations of triglycerides, cholesterol, high density lipoproteins, low density lipoproteins, with enzymatic tests delivered by Roche GmbH (Mannheim, Germany). Free fatty acids and glycerin concentrations were determined by enzymatic tests from Randox GmbH (Crumlin, UK).

### (O) Analysis of insulin

Serum insulin was quantified by an ELISA for rat or mouse insulin that was delivered by CrystalChem (Chigago, USA).

### (P) Analysis of glucose tolerance

The mice were starved for 16 h over night but had access to water. In the morning the body weight and basal D-glucose concentration in the serum were determined. A plastic tube was introduced into the esophagus and 2.5 mg D-glucose per g body weight, dissolved as 1.1 M solution in water, was applied within one min. 10, 20, 60 and 120 min later blood samples were taken from the tail vein and the D-glucose concentrations were analysed.

### (Q) Measurement of intestinal glucose absorption

D-glucose absorption from total small bowel was measured by non-recirculating joint perfusion of isolated small bowel and liver. These organs were perfused via the coeliac trunc and the superior mesenteric artery (SMA) and intestinal glucose absorption was measured from the increase of portal glucose concentration and portal flow rate. The preparation was performed as previously described for the rat (Stümpel et al., Gaastroenterology 110 (1996), 1863-1869). After anaesthesia by intraperitoneal injection of pentobarbital (20 µg/g body weight) the abdomen was opened. A canula was introduced into the SMA, the inferior vena cava cut open and perfusion was started at a hydrostatic pressure of 120 cm H20 and a flow rate of 8 ml/min. The perfusion solution (PSL) was Krebs-Henseleit buffer containing 5 mM D-glucose, 2 mM lactate, 0.2 mM pyruvate, 1 mM glutamine, 3% (w/v) dextran and 1% (w/v) bovine serum albumin. It was equilibrated with a gas mixture of O2/CO2 (19:1). A cannula was introduced via the thoracic aorta into the coleliac trunk and also perfused with PSL using a hydrostatic pressure of 120 cm H2O and a flow rate of 5 ml/min. To facilitate vascular outflow from the liver, a cannula was also introduced through the right atrium in the inferior vena cava with the tip at the inflow of the hepatic veins. For application of an intestinal glucose bolus a catheder was placed through the pylorus in the duodenal lumen. The caecum was incised and the intestinal content washed out with a warmed saline solution. Two flexible small catheders were introduced into the portal vein: one to collect samples for determinationsof glucose concentrations and to measure the portal efflux and the other for infusion of insulin to the liver. Finally, the intestine and liver were carefully prepared free from the body and transferred into an organ bath filled with a warmed saline solution. After a preperfusion period of 20 min the experiment was started by duodenal infusion of 0.25 ml 0.9% NaCl solution containing 100 mg D-glucose within 1 min. Thereafter every minute samples were collected from the portal vein. Later insulin was infused into the portal vein and a second bolus of D-glucose was applied. The flow in the SMA, the efflux from the inferior vena cava and the intestinal effluate were determined as described (Stümpel et al., 1996). The intactness of the preparations was verified throughout all experiments (Stümpel et al., 1996).

### (R) Statistical analysis

Staining reactions in Northern blots and Western blots were quantified by densitometry as described (Korn et al., 2001). Values are given as means +/- SE. The two-tailed unpaired Student t test was used to assess significance of difference between mean values. Linear regression analysis and comparison of slopes was performed with the PRISM progam of GraphPad software Inc. (San Diego, USA). In experiments performed with equally mixed genders it was verified that no gender differences were detectable for the observed parameters.

### Example 2

### Cloning of RS1 from mouse

The RS1 gene from mouse contains an open reading frame coding for 582 amino acids with a relative molecular mass of 61 247 (Genbank accession no. Y11917). It is intronless like the human RS1 gene that is localized an chromosome 1p36.1 (Lambotte et al., DNA and Cell Biology 15 (1996), 769-777). The amino acid sequence of mouse RS1 is 58, 57 and 54 % identical to RS1 from human, pig and rabbit, respectively. The 46 N-terminal amino acids of mouse RS1 contain a consensus sequence for an ubiquitin associated (UBA) domain that is conserved in the four species. Binding of monomeric ubiquitin to this domain has been demonstrated (c.f. Example 13 below). Interestingly, the UBA domain of RS1 contains two dileucine motifs (AS 570,571 and AS 573,574 of mouse RS1) and one consensus sequence of casein kinase II (AS 551 of mouse RS1). Other conserved consensus sequences in RS1 suggest phosphorylation by casein kinase II at amino acids 111 and 329, and by protein kinase C at amino acids 351 and 381 of mouse RS1.

### Example 3

### Targeted disruption of RS1

The coding region of mouse RS1 gene was replaced by an inverted neomycin cassette via homologous recombination in ES cells (Figure 1). Correct targeting of the RS1 allele in ES cells was verified by Southern analysis. From two independent ES clones mice were generated that were heterozygous and homozygous for RS1 disruption as determined by Southern analysis with genomic DNA (Figure 1b). Northern blots with mRNAs isolated from small intestine showed hybridization at 4.4 kb with wild-type and no hybridizition with RS1-/- (Figure 1c). Consistenly, in Western blots performed with a plasma membrane enriched (PME) membrane fraction from small intestine (Figure 1d) RS1 protein could be detected in wild-type but not in RS1-/-. In SDS polyacrylamide gels performed in the presence of mercaptoethanol, mouse RS1 protein showed an atypical slow migration at about 100 kDa as has been decribed for porcine RS.

### Example 4

### Distribution of RS1 protein in small intestine of wild-type mice

The peptide antibody generated against mouse RS1 (RS1 ab) was used to determine the localization of RS1 in small intestine.
Figures 2a-d show that in small intestine RS1 is expressed in epithelial and subepithelial cells. In both cell types RS1 is localized within the nuclei. The nuclear localization was verified by parallel staining of the nuclei with DAPI. In small intestine RS1 was also localized below the plasma membrane of the enterocytes. The immunereaction with RS1 ab was specific. It could be blocked with antigenic peptide (Figure 2e) and was not observed in RS1-/- mice (Figure 2f). The dual localization of RS1 below the plasma membrane and within the nucleus is consistent with the localization of porcine RS1 in the renal epithelial cell line LLC-PK.

### Example 5

### Phenotype analysis of RS1-/- mice

Analysis of heterologous crosses showed distribution of the three genotypes according to Mendelian inheritance (211 RS1+/-, 87 RS1+/+, 95 RS1-/-), indicating that there is no reduced embryonic viability. RS1-/- mice were fertile. They were a little larger in size and heavier in weight compared with their wild type littermates. A striking difference was the increase of abdominal fat in RS1-/- (Figure 3). A comparison between female RS1+/+ and RS1-/- mice at the age of 5 months revealed weights of 22.4 ± 0.3 g and 29.2 ± 0.4 g, respectively. The difference was highly significant (P<0.001). Similar results were obtained with 5 months old male mice indicating no gender differences for the effect of RS1 an weight (RS1+/+ 32.3 ± 0.9 g vs. RS1-/- 38.2 ± 0.9 g, n=10, P < 0.01). In Table 1 the composition of body mass was compared in another group of 5 months old female RS1+/+ and RS1-/- mice (n=5).

**Table 1**

| | | | | | |
|---|---|---|---|---|---|
| Comparison of body composition in female RS1+/+ and RS1-/- mice at the age of 5 months | | | | | |

| | Mass (g) | | | | |
|---|---|---|---|---|---|
| | Total body | Body water | Dry body | Fat | Lean body |
| Wild-type | 24.6 ± 0.7 | 16.7 ± 0.7 | 7.9 ± 0.2 | 1.5 ± 0.1 | 6.4 ± 0.2 |
| RS1(-/-) | 30.8 ± 1.5** | 20.3 ± 1.7* | 10.5 ± 0.7** | 2.7 ± 0.5* | 7.8 ± 0.9* |

Female individual mice were weighed (total body), dried and weighted again (determination of dry body water and water content). The lipids were extracted and the residual dried and weighted again (determination of lean body and fat). Means ± SE, n=5, *P<0.05, **P<0.01

In the RS1-/- mice total body mass was increased by 25 ± 7% (P<0.01), water content by 22 ± 3%(P<0.05), fat content by 80 ± 34% (P<0.05) and fat free dried body mass (lean body) by 22 ± 13 % (P<0.05). By far the largest relative changes were observed in fat content, however, changes in water content and lean body contribute significantly to the changes in total body weight. To evaluate whether the changes in fat content are due to an increase of number or size of the fat cells, the fat cell volume was estimated from electronmicrographs of abdominal fat tissue obtained from 3 female RS1+/+ and RS1-/- mice (see e.g. Figures 3b,c). The relative fat cell volume of RS1+/+ compared to RS1-/ mice was estimated from the section areas of 405 or 480 fat cells an 10 sections, respectively. The fat cell volume in RS1-/- mice was 40 ± 6 % larger compared to wild-type (P<0.01 for difference). The data suggest that the increase of body fat is partially due an increase of fat cell size.

### Example 6

### Feeding behaviour and motoric activity

To determine whether the observed adipositas can be explained by a higher food intake or reduced motor activity in the RS1-/- mice the food intake of 5 months old mice was determined over a period of 7 or 9 days. During the experiments the animals had access to water. Standard laboratory diet ad libitum was either supplied over the whole experiment (Figure 5a) or for 8 h during the day (Figure 5b). This second nonphysiological conditions was tested to detect changes in adaptation. Figure 5 shows identical food intake by RS1+/+ and RS1-/- mice under both condition. The mice needed three days for adaptation to food supply during the day. Thereafter both RS1+/+ and RS1-/- mice showed a significantly reduced uptake compared to unrestricted food supply. Motoric activity was estimated in running wheels where the rotations in both directions were summed up. With this setting no significant change in motoric activity was detected in 5-7 months old RS1-/ mice that were fed ad libitum. In a period of 24 h, RS1+/+ and RS1-/- mice ran 3.30 ± 0.22 and 3.26 ± 0.23 km within the wheels, respectively (n=10, equally mixed gender ).

### Example 7

### Concentrations of D-glucose and lipids in serum

Serum concentrations of D-glucose and some compounds of lipid metabolism were compared in female RS1+/+ and RS1-/- mice at the age of 5 months (n=10). The mice were fed ad libitum and blood was taken in the morning. Between RS1-/- and mice RS1+/+, respectively, no significant different concentrations (mM) were obtained from D-glucose 10.4 ± 0.6 vs. 10.7 ± 0.5, triglycerides 1.3 ± 0.1 vs. 1.3 ± 0.1, high density lipid proteins 2.1 + 0.1 vs 1.8 + 0.2, low density lipoproteins 0.27 ± 0.03 vs. 0.25 ± 0.02 , free fatty acids 0.89 ± 0.06 vs. 0.82 ± 0.05 and glycerol 0.36 ± 0,03 vs. 0.39 ± 0.02. At variance the serum cholesterol concentration in RS1-/- mice was signiciantly higer compared to wild-type (3.0 ± 0.2 mM vs. 2.3 ± 0.2 mM, P<0.05) .

### Example 8

### Regulation of serum glucose and glucose reabsorption in small intestine

Since previous in vitro experiments showed that RS1 is involved in the regulation of the Na⁺-D-glucose cotransporter SGLT1 and the activity of SGLT1 is increased by insulin the postprandial regulation of serum D-glucose was investigated. A glucose tolerance test was performed with 5 months-old mice that had been starved over night. The serum concentrations of D-glucose in the morning were 7.7 ± 0.2 and 7.9 ± 0.2 mM for RS1+/+ and RS1-/-, respectively (n=10). A bolus of 2.5 mg D-glucose per g body weight was applied as 1.1 molar solution to the esophagus of non-anaesthesized mice. After 10 min serum D-glucose was increased 87 ± 12% in RS1+/+ mice and 103 ± 11% in RS1-/- mice (Figure 6a). Although the difference in D-glucose increase was not significant the possibility was considered that it could indicate a change of intestinal D-glucose reabsorption that was obscured by glucose uptake into the liver (see below). In RS1-/- and RS1+/+ mice a similar down-regulation of the postprandial D-glucose increase in the serum was observed (Figure 6a). After 24 hours of starvation the serum insulin was significantly decreased in wildtype and RS1-/- mice (Figure 6b). The serum insulin concentration in wildtype and RS1-/- mice were not significantly different. The data suggest an insulin dependent downregulation of serum D-glucose, and insulin secretion in the pancreas are not largely disturbed in RS1-/- mice.

The small intestinal reabsorption of D-glucose was measured employing a perfused ex vivo preparation of total small intestine and liver that has been previously employed in rat. Oxygen and metabolic fuels including 5 mM D-glucose were supplied by noncirculating perfusion of small intestine and liver via the coeliac trunc and the superior mesenteric artery allowing venous outflow through the vena cava. Glucose absorption by total small intestine was determined by infusing a bolus of 100 mg D-glucose dissolved in 0.25 ml saline and measuring the increase of the D-glucose concentration in the portal vein (Figure 7a). Measuring the outflow of the portal vein collecting all intestinal veins, the total amount of absorbed D-glucose could be determined (Figure 7b). Using this model in the rat it was previously observed that small intestinal D-glucose absorption was increased when insulin was applied to the portal vein. This upregulation is supposed to be mediated by stimulation of β-adrenergic nerves since insulin applied to the portal vein does not reach the small intestine. Under basal and stimulated conditions, the absorption of D-glucose in small intestine was significantly higher in RS1-/- mice compared to wild-type. In RS1-/- mice compared to wild-type the total amount of absorbed D-glucose was increased by 117 ± 43% (basal) or 94 ± 13% (insulin). The data suggest that in RS1-/- mice Na⁺-D-glucose cotransport activity is increased whereas the upregulation of glucose absorption by insulin is not effected.

### Example 9

### Expression of SGLT1 and GLUT2 in small intestine

Figure 8 shows the immunohistological distribution of the Na⁺ -D-glucose cotransporter SGLT1 in jejunum of RS1+/+ and RS1-/- mice. The immunoreaction was performed with the affinity purified antibody SGLTlab that was raised against a subtype specific peptide. The brush-border membranes of the enterocytes were stained and the reactions could be blocked with the antigenic peptide In RS1-/- mice the immunoreaction with SGLTlab was much stronger compared to wild type (Figure 8a,b). Laser scanning micrographs showed that SGLT1 immunoreactivity was observed at and below the brush-border membrane and that it was increased in RS1-/- mice at both locations. To quantify the increase of SGLT1 Western blots were performed with plasma membrane enriched (PME) membrane fractions from small intestine (Figure 8c,d). SGLTlab reacted with a single band at about 70kDa and the reaction could be blocked with antigenic peptide. Western blots prepared from RS1-/- mice showed a much stronger reaction with SGLT1 ab. A densitometric quantification of 6 reactions from 2 independent experiments blots revealed a 7-fold higher amount of SGLT1 protein in RS1-/- (P<0.001 for difference). In LLC-PK1-cells previously it was observed that the transcription of SGLT1 was increased when the intracellular concentration of RS1 was reduced by an antisense strategy. To determine whether the increase of SGLT1 protein in RS1-/- mice may be explained by upregulation of transcription we determined whether SGLT1 mRNA was increased. In Northern blots no significant differences between RS1-/- and RS1+/+ were detected for the 3.9 and 2.2 kb transcripts of the SGLT1 gene (Fig. 8e,f). Similar transcripts of the SGLT1 gene that differ in length of the 3'untranslated region have been observed in pig. The data indicate that the upregulation of SGLT1 in RS1-/- mice is due to posttranslational changes. This is at variance to the changes observed in LLC-PK1 cells.

In addition to SGLT1 the passive D-glucose transporter GLUT2 is required for D-glucose absorption in small intestine. GLUT2 is expressed in the basolateral membrane and it mediates D-glucose efflux out of the enterocytes. If the intestinal lumen contains high D-glucose concentrations GLUT2 may undergo rapid trafficking from the basolateral to the brush border membrane and may participate in luminal glucose uptake. To determine whether GLUT2 is upregulated in RS1-/- mice the expression of GLUT2 in the PME fraction of the enterocytes that contains luminal and basolateral plasma membranes was inverstigated. The Western blots in Figure 9 were developed with a subtype specific antibody against GLUT2 obtained from Alpha Diagnostic International (San Antonio, USA). The antibody reacted mainly with a 60 kDa band and showed a weak reaction at 52 kDa. Both reactions could be blocked with antigenic peptide. Densitometric quantification of Western blots revealed no significant difference between GLUT2 in RS1-/- and RS1+/+ (Figure 9b). Similarly, Northern blots that were developed with a specific cDNA probe for GLUT2 showed no significant difference between GLUT2 mRNA in RS1-/- compared to wild-type (data not shown). The data indicate that the upregulation of D-glucose absorption in RS1-/- is only due to upregulation of SGLT1.

### Example 10

### Tissue specificity for upregulation of SGLT1 in RS1-/- mice

Similar to enterocytes, RS1 and SGLT1 are also expressed in renal proximal tubules. SGLT1 protein and SGLT1 mRNA in kidneys of RS1+/+ and RS1-/- mice were compared. In Western blots of renal brush border membranes that were developed with SGLT1 ab no significant differences could be detected between RS1+/+ and RS1-/- mice. The same result was obtained when kidney sections were immunostained with SGLT1 ab (data not shown). Similarly, no significant differences in SGLT1 mRNAs were detected in Northern blots that were prepared from kidneys of RS1+/+ and RS1-/-mice. The data indicate that the effects of RS1 on the expression of SGLT1 in small intestine is tissue specific.

### Example 11 (For illustration only)

### Demonstration that hRS1 increases dynamin dependent endocytosis.

- ***Methods***
- *Plasmids for expression in oocytes:* Plasmids containing hRS1 and hSGLT1 were prepared as described earlier (Lambotte et al., (1996) DNA Cell Biol. 15, 769-777; Wells et al., (1992) Am. J. Physiol. 263:F459-465). DNA of rat wildtype dynamin (DyWt) and dominant-negative mutant of dynamin (DyMu) (Sontag et al. (1994) J. Biol. Chem. 269, 4547-4554) were digested with *SspI* and *KpnI* and cloned into the oocyte expression vector pOG2 cut with SmaI and *KpnI* (Arndt et al. (2001) Am. J. Physiol. Renal. Physiol. 281, F454-F468.)
- *Plasmids for expression in HEK293 cells:* For ubiquitin binding studies porcine RS1 (pRS1) in Bluescript II SK plasmid was restricted with BglII and HindIII and cloned into the *BstX* I sites of the eucaryotic expression vector pRcCMV from Invitrogen, Leek, Netherlands (pRcCMV-pRS1) as described (Korn et al. (2001) J. Biol. Chem. 276,45330-45340). To create C-terminal truncated pRS1 without ubiquitin binding domain in pRcCMV (pRcCMV-pRS1Δ511-623), the plasmid pRcCMV-pRS1 was digested with *Alw*441, the ends filled up using Klenow fragment of DNA polymerase I, and the plasmid digested with *Hind*III. The N-terminal pRS1-fragment was isolated and cloned into the pRcCMV vector that was cut with *XbaI* and *Hind*III.
- In vitro synthesis of cRNA for expression in oocytes
   For injections into Xenopus oocytes 5'7meGppp5'G capped cRNA was prepared, purified, evaluated and stored as described earlier (Veyhl et al., (1994) J. Biol. Chem. 268, 25041-25053). To prepare sense cRNA from hRS1 (Lambotte et al., (1996) DNA Cell Biol. 15, 769-777 ), hSGLT1 (Wells et al., (1992) Am. J. Physiol. 263:F459-465), wild-type (DyWT) and mutant dynamin (DyMu), the purified plasmids were linearized with EcoRI (hSGLT1) and XbaI (hRS1). The cRNA was synthesized by T3 polymerase (hSGLT1), T7 polymerase (hRS1, DyWt, DyMu) and linearized with *XbaI* (hRS1), EcoRI (hSGLT1) or NotI (DyWt, DyMu).
- Expression of plasma membrane transporters in oocytes of Xenopus laevis
   Stage V-VI oocytes were removed from Xenopus laevis clawed toads, selected and injected with cRNAs or water as described earlier (Veyhl et al., (1994) J. Biol. Chem. 268, 25041-25053). Per oocyte 50 nl of water (control) or water containing plasma membrane transporter cRNAs alone or together with different amounts of hRS1-cRNA were injected. For translation the injected oocytes were incubated for 2-3 days (16°C) in ORi buffer (5mM HEPES-Tris, pH 7.4, 100 mM NaCl, 3mM KCl, 2 mM CaCl and 1 mM MgCl₂) containing 50 mg/l gentamycin (Veyhl et al., (1994) J. Biol. Chem. 268, 25041-25053).
- Transport measurements in oocytes of Xenopus laevis
   [¹⁴C]-AMG uptake of expressed plasma membrane transporters in oocytes was measured as described earlier Veyhl et al., (1994) J. Biol. Chem. 268, 25041-25053. Each uptake value represents the mean ± SEM (n = 8-10 oocytes). All experiments were performed with at least 3 batches of Xenopus oocytes.
- Results
   Tracer uptake studies and electrical measurements showed that the Vₘₐₓ of AMG transport by hSGLT1 is decreased after coexpression with hRS1 and that hRS1 does not change the Kₘ value of AMG uptake expressed by hSGLT1 as previously assumed (unpublished data, not shown). It has been investigated whether the decrease in transport activity of hSGLT1 observed after expression of hRS1 is caused by internalisation of transporter from the plasma membrane via dynamin-dependent endocytosis. Dynamin, a member of structurally related but functionally heterogeneous family of GTPases, that is required for clathrin-mediated and other types of endocytosis. After GTP binding dynamin assembles to a ring around the neck of coated pits and induces fission and release of vesicles upon hydrolysis of GTP (Eccleston et al., (2002) Eur. Biophys. J. 31, 275-282.). Mutations that disrupt the GTP-binding domain such as lysine 44 of dynamin in the employed dominant negative dynamin mutant lead to a loss of GTPase activity and to inhibition of both clathrin- and caveolin-mediated endocytosis (Damke et al. (1994) J. Cell. Biol. 127, 915-934; Hinshaw (2000) Annul. Rev. Cell Dev. Biol. 16, 483-519.) To specifically block endocytosis, SGLT1 or hSGLT1 plus hRS has been coexpressed with either wild-type dynamin or with the dominant negative dynamin mutant. Fig. 10 shows a representative experiment out of four. Oocytes were injected with 2.5 ng of hSGLT1-cRNA or with 2.5 ng pf hSGLT1-cRNA plus 10 ng of hRS1-cRNA. In some experiments either 10 ng of wild-type (DyWt) dynamin cRNA or 10 ng of dominant gegative dynamin mutant (DyMu) cRNA were coinjected. After three days of incubation for expression, phlorizin inhibitable uptake of 50 µM of [¹⁴C]AMG was measured over a time period of 15 min as described earlier (Lambotte et al., (1996) DNA Cell Biol. 15, 769-777). In oocytes injected with hSGLT1 plus DyWt glucose transport of similar magnitude was observed compared to oocytes that were only injected with hSGLT1((pmol/oocyte x 15 min) hSGLT1: 56 ± 7, hSGLT1 + DyWt: 58 ± 8, hSGLT1 + DyMu: 51 ± 5). In oocytes injected with hSGLT1-cRNA together with hRS1-cRNA the typical inhibition of transport activity by hRS was observed (10 ± 1.5 pmol/ oocyte x 15 min). In contrast the hRS1 effect on hSGLT1 was partially blocked by coinjection of the dynamin mutant (37 ± 3.5 pmol / oocyte x 15 min) compared to oocytes expressing wild-type dynamin (2.5 pmol / oocyte x 15 min). In two of four experiments the reduction of hSGLT1 mediated transport by hRS1 was strengthened in oocytes coinjected with wild-type dynamin suggesting that the amount of endogeneous active dynamin is a rate limiting step in hRS1 overexpressing oocytes. The data indicate that the reduction of Vₘₐₓ of hSGLT1 results from removing of transport protein from the plasma membrane by dynamin dependent endocytosis induced by hRS1 (Figure 10).

### Example 12 (For illustration only)

### Demonstration that protein kinase C activation leads to an activation of hRS1.

According to the methods described in Example 11 *Xenopus* oocytes were injected with 2.5 ng of SGLT1-cRNA or with 2.5 ng of SGLT1-cRNA plus 10 ng of hRS1 and incubated three days for expression (Fig. 11). Then the oocytes were injected with 50 nl Ori buffer (controls) or with 50 nl Ori buffer containing 20 µM of the protein kinase C activator sn-1,2-dioctanoyl glycerol (DOG). After 30 min incubation the phlorizin inhibitable uptake of 50 µM [¹⁴C]AMG was measured as in Fig. 10. Fig. 11 shows a representative experiment out of three. By DOG, the uptake of AMG expressed by hSGLT1 was significantly (*P<0.01) increased as described in the literature (Hirsch et al. (1996) J. Biol. Chem. 271, 14740-14746). The figure shows also that the expression of AMG uptake by hSGLT1 was reduced after coexpression of hRS1 (**P<0.01). Notably, DOC has a different effect on the expressed uptake when hSGLT1 and hRS1 were coexpressed compared to the expression of hSGLT1 alone. After coexpression of hSGLT1 and hRS1 the expressed uptake of AMG was significantly (*P<0.01) decreased by DOG rather than increased as observed after expression of hSGLT1 alone. The data indicate that protein kinase C increases the effect of hRS1 on SGLT1 (Figure 11). This opens the possibility that the action of RS1 can be modilated by activators and inhibitors of protein kinase C.

### Example 13 (For illustration only)

### Non covalent binding of ubiquitin monomer to pRS1

- **Methods**
- *Cell Culture and Transfection of epithelial cells*
   Wild-type and transfected cells of the the human embryonal kidney (HEK) cell line 293 were maintained in Dulbecco's Modified Eagle's Medium (DMEM) that was supplemented with 10% (v/v) fetal bovine serum, 5 mM L-glutamine, 0.1 mg/ml streptomycin sulfate and 100 U/ml penicillin G. Cells were grown at 37°C on Petri dishes in the presence of 5% (v/v) CO₂ and the culture medium was replaced every 2 to 3 days. For passage, the HEK 293 cells were detached mechanically using a pipette, aspirated, pelleted by 5 min centrifugation at 250 x g and resupended in culture medium. For transient transfection of pRS1 and pRS1Δ511-623 the FuGene6 transfection reagent from Boehringer (Mannheim, Germany) was used. 48 h after transfection, the HEK 293 cells were analysed for binding to ubiquitin-agarose.
- *Binding of pRS1 to ubiquitin agarose*
   HEK 293 cells were incubated 15 min at 4°C with 1% (v/v) Igepal CA630 (Sigma, Münchenm) dissolved in 25 mM Tris-HCl pH 7.5, 100 mM NaCl containing 1 mM benzamidin, 5 µg/ml aprotinin, 5 µg/ml leupeptine and 1 mM phenylmethylsulfonylfluride. The detergent treatment leads to lysis of the cells and to a dissociation of pRS1 from renal brush-border membranes. After cell debris and aggegated material was removed by 10-min centrifugation at 6 000 x g, ubiquitin was removed from the supernatant by ion exchange chromatography as described (Vadlamudi et al. (1996) J. Biol. Chem. 271, 20235-20237). pRS1 and other acidic proteins were bound to DEAE-Sephadex by incubating the supernatant for 1 h at 4° with the same volume of DEAE-Sephadex A25-100 beads (Sigma) that had been equilibrated with 25 mM Tris-HCl pH 7.5, 100 mM NaCl (TrisNa buffer). The beads were filled into a small column, washed extensively with TrisNa buffer. The acidic proteins including pRS1 were eluted with 25 mM TrisHCl pH 7.5, 1 mM KCl and collected in a volume of 50 µl (Ub-free lysate). To test pRS1 for binding to ubiquitin, 25 µl of the Ub-free lysate containing about 50 µg of protein were diluted with 225 µl of TrisNa buffer and 50 µl of ubiquitin-agarose beads from Calbiochem (Bad Soden, Germay) that had been equilibrated with TrisNa buffer, were added. The mixture was shaked for 2 h at 4°C. Then the beads were washed 2 times by incubation with 10 volumes of TrisNa buffer and 6000 x g-centrifugation, and bound proteins were eluted from the beads with 50 µl of 46 mM Na₂HPO₄, 54 mM Na₂ H₂PO₄ pH 6.8, 2% (w/v) SDS and 0.5 M β-mercaptoethanol containing 0.001% (w/v) bromphenol blue (2x sample buffer of SDS-poyacylamide gel electrophoresis ).
- Results:
   RS1-proteins from pig, mouse and human contain an ubiquitin-associated (UBA) domain at the C-terminus that is 100% conserved in these species (Valentin et al. (2000) Biochim. Biophys. Acta 1468, 367-380). UBA domains have been described in many proteins and have been associated with a variety of functions as regulation of protein degradation in the proteasome, regulation of transcription in nuclei and regulation of exo- or endocytosis (Hofmann and Bucher (1996), Trends Biochem. Sci. 21, 172-173; Hicke (2001) Nat. Rev. Mol. Cell. Biol. 2,195-201). For the UBA domains of some proteins ubiquitin binding has been demonstrated. Trying to understand the role of the UBA domain in RS1 we tested whether porcine RS1 (pRS1) binds to ubiquitin and whether this binding is due to the UBA domain of RS1.

In Fig. 12a) porcine RS1 (pRS1) and b) pRS1 without the C-terminus containing the UBA domain (pRS1Δ583-623) were transiently expressed in HEK 293 cells. Two days after transfection the cells were lysed and cell debris was removed (Lysate). Then endogeneous ubiquitin was removed from the lysates by ion exchange chomatograpy (Ub-free lysate) and 50 µl of this solution containing about 50 µg of protein was incubated with 50 µl ubiquitin agarose beads and washed. Bound proteins were removed with 50 µl sample buffer containing 2% (w/v) SDS (UbAgeluate), separated by SDS polyacrylamide gel electrophoresis and analysed by immunostaining with pRSlab. From the UbAg-eluates 20 µl were applied to lanes 4 and 8. 20 µg of proteins were applied to lanes 1-3 and 5-7. The proteins were blotted to nitrocellulose and immunostained with affinity purified pRS1-ab as described (Korn et al.(2001) J. Biol. Chem. 276, 45330-45340). In lanes 1 and 5 porcine renal brush-border membranes were (BBM) were applied as controls.

The data indicate that pRS1 can be affinity purified with monoubiquitin that has been covalently linked to agarose. If the ubiquitin-associated domain was removed no affinity purification via the interaction of pRS1 and ubiquitin was detected. Control experiments with unmodified agaose beads (without ubiquitin) showed no affinity purification of pRS1 (data not shown). The data indicate that the UBA domain of RS1 binds monoubiquitin.

## Claims

1. A transgenic mouse homozygous for a targeted deletion of the coding region of the gene encoding the regulatory subunit (RS1) of sodium glutamate co-transporter SGLT1, **characterized in that** it shows increased body weight, increased total fat and increased mean fat cell volume compared to a wild type mouse.

2. The transgenic mouse of claim 1, wherein the gene encoding RS1 contains a deletion within its coding region.

3. The transgenic mouse of claim 2, wherein the entire coding region of the gene encoding RS1 has been deleted.

4. Use of the transgenic mouse of any one of claims 1 to 3 as an animal model for obesity or hypercholesterolaemia.

5. Use of the transgenic mouse of any one of claims 1 to 3 for identifying substances suitable for the therapy and/or prevention of obesity or hypercholesterolaemia.

6. Use of the transgenic mouse of any one of claims 1 to 3 for assaying the efficiency of dieting or pharmacological therapy of obesity or hypercholesterolaemia.

7. Use of
a RS1 encoding nucleic acid molecule
for the preparation of a pharmaceutical composition for treatment of obesity or hypercholesterolaemia.

8. A method for the diagnosis of obesity or hypercholesterolaemia or of a risk for developing said diseases, comprising the following steps:
(a) contacting a sample with an anti-RS1-antibody capable of specifically binding to RS1 or with a probe comprising at least one oligonucleotide capable of specifically hybridizing to RS1DNA or mRNA; and
(b) determining the concentration and/or sequence of RS1 or the RS1 DNA or mRNA
wherein a decreased concentration of RS1 or RS1 mRNA or the presence of a mutation within RS1 DNA or mRNA sequence resulting in an RS1 protein with complete or partial loss of activity is indicative for obesity or hypercholesterolaemia or for a risk for developing said diseases, compared to healthy individuals.

9. The method of claim 8, wherein the anti-RS1-antibody or the at least one oligonucleotide is detectably labeled.

## Patentansprüche

1. Transgene Maus, die für eine gezielte Deletion der Codierregion des Gens, das für die Regulationsuntereinheit (RS1) des Natriumglutamat-Cotransporters SGLT1 codiert, homozygot ist, **dadurch gekennzeichnet, daß** sie ein im Vergleich zu einer Wildtypmaus erhöhtes Körpergewicht, erhöhtes Gesamtfett und erhöhtes mittleres Fettzellenvolumen aufweist.

2. Transgene Maus nach Anspruch 1, wobei das für RS1 codierende Gen eine Deletion innerhalb seiner Codierregion enthält.

3. Transgene Maus nach Anspruch 2, wobei die gesamte Codierregion des für RS1 codierenden Gens deletiert worden ist.

4. Verwendung der transgenen Maus nach einem der Ansprüche 1 bis 3 als Tiermodell für Fettleibigkeit oder Hypercholesterolämie.

5. Verwendung der transgenen Maus nach einem der Ansprüche 1 bis 3 zum Identifizieren von Substanzen, die sich für die Therapie und/oder Prävention von Fettleibigkeit oder Hypercholesterolämie eignen.

6. Verwendung der transgenen Maus nach einem der Ansprüche 1 bis 3 zum Testen der Wirksamkeit des Einhaltens einer Diät oder einer pharmakologischen Therapie für Fettleibigkeit oder Hypercholesterolämie.

7. Verwendung eines für RS1 codierenden Nukleinsäuremoleküls für die Herstellung einer pharmazeutischen Zusammensetzung für die Behandlung von Fettleibigkeit oder Hypercholesterolämie.

8. Verfahren für die Diagnose von Fettleibigkeit oder Hypercholesterolämie oder des Risikos des Entstehens dieser Krankheiten, umfassend die folgenden Schritte:
(a) Inkontaktbringen einer Probe mit einem anti-RS1-Antikörper, der spezifisch an RS1 zu binden vermag, oder mit einer Sonde umfassend mindestens ein Oligonukleotid, das spezifisch mit RS1-DNA oder -mRNA zu hybridisieren vermag; und
(b) Bestimmen der Konzentration und/oder Sequenz von RS1 oder der RS1-DNA oder -mRNA,
wobei eine verringerte Konzentration an RS1 oder RS1-mRNA oder das Vorliegen einer Mutation innerhalb der RS1-DNA- oder -mRNA-Sequenz, die zu einem RS1-Protein mit vollständigem oder teilweisem Aktivitätsverlust führt, Fettleibigkeit oder Hypercholesterolämie bzw. das Risiko des Entstehens dieser Krankheiten im Vergleich zu gesunden Probanden anzeigt.

9. Verfahren nach Anspruch 8, wobei der Anti-RS1-Antikörper oder das mindestens eine Oligonukleotid mit einem nachweisbaren Marker versehen ist.

## Revendications

1. Souris transgénique homozygote pour une délétion ciblée de la région codante du gène codant pour la sous-unité régulatrice (RS1) du cotransporteur sodium-glutamate SGLT1, **caractérisée en ce qu'**elle affiche une augmentation du poids corporel, une augmentation des graisses totales et une augmentation du volume moyen des cellules adipeuses par rapport à une souris de type sauvage.

2. Souris transgénique selon la revendication 1, selon laquelle le gène codant pour RS1 contient une délétion dans sa région codante.

3. Souris transgénique selon la revendication 2, selon laquelle la région codante entière du gène codant pour RS1 a été délétée.

4. Utilisation de la souris transgénique selon l'une quelconque des revendications 1 à 3 comme modèle animal pour l'obésité ou l'hypercholestérolémie.

5. Utilisation de la souris transgénique selon l'une quelconque des revendications 1 à 3 pour l'identification de substances appropriées pour la thérapie et/ou la prévention de l'obésité ou de l'hypercholestérolémie.

6. Utilisation de la souris transgénique selon l'une quelconque des revendications 1 à 3 pour évaluer l'efficacité d'une diète ou d'une thérapie pharmacologique pour l'obésité ou l'hypercholestérolémie.

7. Utilisation d'une molécule d'acide nucléique codant pour RS1 pour la préparation d'une composition pharmaceutique destinée au traitement de l'obésité ou de l'hypercholestérolémie.

8. Méthode permettant le diagnostic de l'obésité ou de l'hypercholestérolémie ou d'un risque de développer lesdites maladies, comprenant les étapes suivantes :
(a) mettre en contact un échantillon avec un anticorps anti-RS1 susceptible de se lier spécifiquement à RS1 ou avec une sonde comprenant au moins un oligonucléotide susceptible de s'hybrider spécifiquement à l'ADN ou l'ARNm de RS1 ; et
(b) déterminer la concentration et/ou la séquence de RS1 ou de l'ADN ou l'ARNm de RS1 selon laquelle une baisse de la concentration de RS1 ou de l'ARNm de RS1 ou la présence d'une mutation dans la séquence d'ADN ou d'ARNm de RS1 conduisant à une protéine RS1 présentant une perte d'activité complète ou partielle est indicatrice d'une obésité ou d'une hypercholestérolémie ou d'un risque de développer lesdites maladies, par rapport à des individus sains.

9. Méthode selon la revendication 8, selon laquelle l'anticorps anti-RS1 ou ledit ou lesdits oligonucléotides sont marqués de façon détectable.


### SEQUENCE LISTING

<110> Koepsell Hermann
<120> RS1 deficient transgenic animal and uses thereof
<130> P43688 EP
<160> 7
<170> PatentIn version 3.1
<210> 1
   <211> 24
   <212> DNA
   <213> pig
<400> 1
   aatccgcctt agatggaagt agga 24
<210> 2
   <211> 22
   <212> DNA
   <213> pig
<400> 2
   gcttcctgca aaggtagaag cc 22
<210> 3
   <211> 25
   <212> DNA
   <213> mouse
<400> 3
   ccccacaccc ttcccatggt catga 25
<210> 4
   <211> 25
   <212> DNA
   <213> mouse
<400> 4
   gggaatgcag accttgccct tcttg 25
<210> 5
   <211> 25
   <212> DNA
   <213> mouse
<400> 5
   ccacttgtgt agcgccaagt gccag 25
<210> 6
   <211> 19
   <212> PRT
   <213> mouse
<400> 6
   Gly Leu Ser Pro Asp Arg Glu Asp Val Arg Arg Ser Thr Glu Ser Ala
   1               5                   10                  15
   Arg Lys Ser
<210> 7
   <211> 16
   <212> PRT
   <213> mouse
<400> 7
   Lys Asp Thr Ile Glu Ile Asp Thr Glu Ala Pro Gln Lys Lys Lys Gly
   1               5                   10                  15